(19) Europäisches Patentamt
European Patent Office
Office européen des brevets

(11) Publication number: **0 028 099**
**B1**

(12) # EUROPEAN PATENT SPECIFICATION

(45) Date of publication of patent specification: **23.11.83**

(51) Int. Cl.³: **G 03 C 7/34,** C 07 C 127/19

(21) Application number: **80303642.5**

(22) Date of filing: **15.10.80**

(54) **Photographic couplers, emulsions, materials and processes.**

(30) Priority: **15.10.79 US 85140**

(43) Date of publication of application:
**06.05.81 Bulletin 81/18**

(45) Publication of the grant of the patent:
**23.11.83 Bulletin 83/47**

(84) Designated Contracting States:
**BE CH DE FR GB IT LI**

(56) References cited:
**DE - A - 2 263 171**
**FR - A - 2 382 437**

**The file contains technical information submitted after the application was filed and not included in this specification**

(73) Proprietor: **EASTMAN KODAK COMPANY**
**343 State Street**
**Rochester New York 14650 (US)**

(72) Inventor: **Lau, Philip Thiam Shin**
**Kodak Park**
**Rochester, New York (US)**

(74) Representative: **Pepper, John Herbert et al,**
**KODAK LIMITED Patent Department P.O. Box 114**
**190 High Holborn**
**London WC1V 7EA (GB)**

**EP 0 028 099 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Photographic couplers, emulsions, materials and processes

This invention relates to novel phenolic cyan dye-forming couplers, to photographic silver halide emulsions and materials containing these couplers and to processes of forming cyan dye images with materials containing these couplers.

Colour images are customarily obtained in the photographic art by reaction between the oxidation product of a silver halide aromatic primary amino colour developing agent and a dye-forming compound known as a coupler. The reaction between coupler and oxidized colour developing agent results in coupling of the oxidized colour developing agent at a reactive site on the coupler, known as the coupling position, and yields a dye. The dyes produced by coupling are indoaniline, azomethine, indamine, or indophenol dyes, depending upon the chemical composition of the coupler and the developing agent. The subtractive process of colour image formation is ordinarily employed in multicolour photographic materials and the dyes produced by coupling are usually cyan, magenta and yellow dyes which are formed in or adjacent to silver halide emulsion layers sensitive to the red, green and blue regions of the spectrum respectively.

The couplers which typically are employed to produce cyan dyes are phenols and naphthols. They yield azomethine dyes upon coupling with oxidized aromatic primary amino colour developing agents.

Phenol couplers containing a ureido group in the 2-position are described in U.K. Patent 1,011,940 and U.S. Patents 3,446,622, 3,996,253, 3,758,308, 3,864,366 (equivalent to DE—A—2,263,171; see especially Table 1 Coupler 9), and 3,880,661. These couplers generally give dyes having good light stability. However, many of such couplers yield dyes having absorption maxima ($\lambda_{max}$) in the shorter wavelength portion of the red region of the spectrum or having relatively broad spectral absorption curves, or both. Thus, such couplers yield dyes having undesirable hues for photographic purposes and frequently having significant absorption in the green region of the spectrum. In addition, a number of the dyes fade when contacted with ferrous ion, and thus, have poor stability in commonly employed processing compositions.

These problems are largely, if not completely, overcome by the use of the couplers of this invention which have the structural formula:

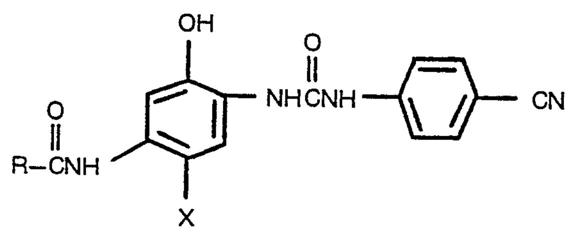

Formula I

wherein:

X is hydrogen or a coupling-off group; and

R is a ballast group.

The couplers of the invention can be used in photographic silver halide emulsions and in photographic materials comprising a support and a silver halide emulsion wherein the coupler is contiguous to the silver halide. Such photographic materials, after imagewise exposure, can be processed to cyan dye images by developing with an aromatic primary amino colour developing agent. The cyan dyes produced have absorption maxima ($\lambda_{max}$) in the longer wavelength portion of the red region of the visible spectrum (generally above 650 nm), and thus, they are dyes of desirable hue for photographic images. The dyes are also desirable through having relatively narrow spectral absorption curves, little absorption in the green region of the spectrum, and good stability to ferrous ions that are commonly found in bleach-fix processing baths.

Coupling-off groups defined by X in Formula I are well known to those skilled in the art. Such groups can determine the equivalency of the coupler (i.e., whether it is a two-equivalent coupler or a four-equivalent coupler), can modify the reactivity of the coupler, and/or can advantageously affect the layer in which the coupler is coated or other layers in the material by performing, after release from the coupler, such functions as development inhibition, bleach inhibition, bleach acceleration and colour correction. Representative classes of coupling-off groups include halogen, alkoxy, aryloxy, heteroyloxy, sulphonyloxy, acyloxy, acyl, heteroyl, thiocyano, alkylthio, arylthio, heteroylthio, sulphonamido, phosphonyloxy and arylazo. Such coupling-off groups are described, for example, in U.S. Patents 2,455,169; 3,227,551; 3,432,521; 3,476,563; 3,617,291; 3,880,661; 4,052,212 and 4,134,766; and in U.K. Patents and Published Applications 1,466,728; 1,531,927; 1,533,039; 2,006,755A and 2,017,704A.

Examples of coupling-off groups are:

—Cl, —F, 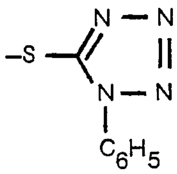, 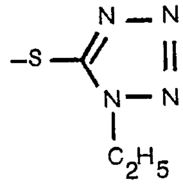 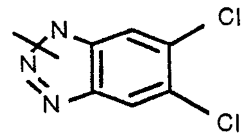,

—SCN, —$OCH_3$, —$OC_6H_5$,

—$OCH_2CONHCH_2CH_2OH$, —$OCH_2CONHCH_2CH_2OCH_3$,

—$OCH_2CONHCH_2CH_2OCOCH_3$,

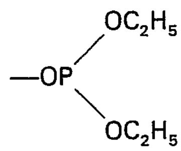

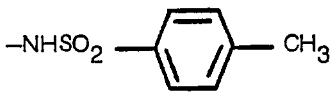 and —$OCH_2CH_2NHSO_2CH_3$.

The ballast group defined by R in Formula I is an organic radical of such size and configuration as to confer on the coupler molecule sufficient bulk to render the coupler substantially non-diffusible from a layer in which it is coated on a photographic material. Representative ballast groups include substituted or unsubstituted alkyl or aryl groups containing a total of 8 to 32 carbon atoms. Representative substituents include alkyl, aryl, alkoxy, aryloxy, alkylthio, arylthio, hydroxy, halogen, alkoxycarbonyl, aryloxycarbonyl, carboxy, acyl, acyloxy, carbonamido, carbamoyl, alkylsulphonyl, arylsulphonyl, sulphonamido, and sulphamoyl groups wherein the alkyl and aryl substituents, and the alkyl and aryl portions of the alkoxy, aryloxy, alkylthio, arylthio, alkoxycarbonyl, arylcarbonyl, acyl, acyloxy, carbonamido, carbamoyl, alkylsulphonyl, arylsulphonyl, sulphonamido and sulphamoyl substituents contain 1—30 carbon atoms and 6 to 30 carbon atoms, respectively, and can be optionally substituted with such substituents.

R in Formula I preferably has the structural formula:

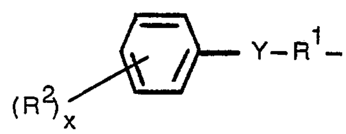

Formula II

wherein:

Y is oxygen or sulphur;

$R^1$ is an alkylidene group of 2 to 20 carbon atoms having the formula

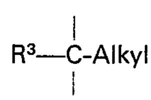

where $R^3$ is hydrogen or alkyl;

$R^2$ is hydroxy, carboxyl, alkyl, aryl, aralkyl, alkoxyl, aryloxy, alkylsulphamoyl, arylsulphamoyl, alkylsulphonamido, arylsulphonamido, alkylsulphonyl, arylsulphonyl, alkoxycarbonyl, or acyloxy wherein alkyl contains 1 to 20 carbon atoms and aryl contains 6 to 20 carbon atoms and wherein alkyl, aryl and aralkyl can be optionally substituted with hydroxy, carboxy, alkoxycarbonyl or acyloxy; and

x is 1 to 3.

Especially preferred are those couplers where $R^2$ is straight or branched chain alkyl of 1 to 20 carbon atoms and x is 1 or 2.

Specific couplers of this invention are shown in Table I below with reference to Formula I above.

TABLE I

| Coupler Number | X | R |
|---|---|---|
| 1 | –H | 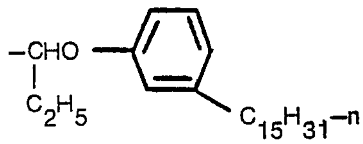 |
| 2 | –Cl | 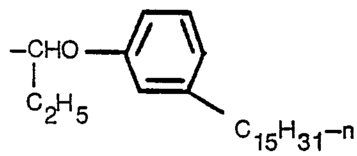 |
| 3 | –H | 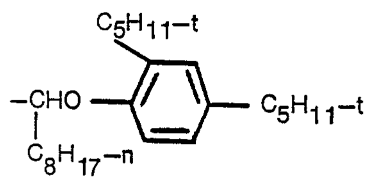 |
| 4 | –H | 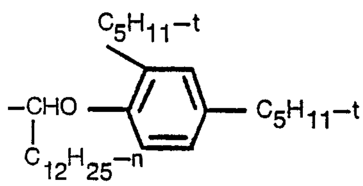 |
| 5 | –H | 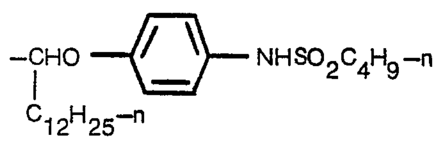 |
| 6 | –H | 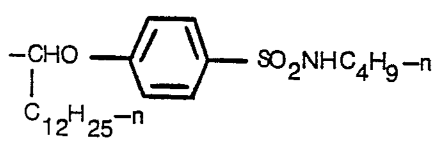 |
| 7 | –H | 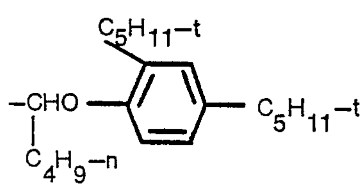 |

TABLE I (Continued)

| Coupler Number | X | R |
|---|---|---|
| 8 | –Cl | 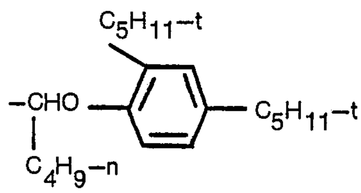 |
| 9 | –H | 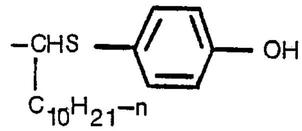 |
| 10 | –H | 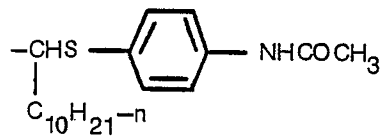 |
| 11 | –Cl | 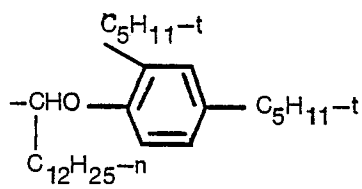 |
| 12 | –F | 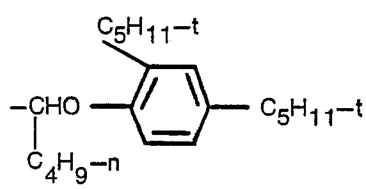 |
| 13 | –H | 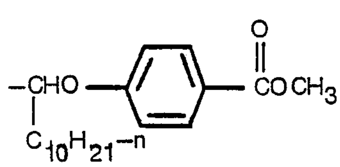 |
| 14 | –H | 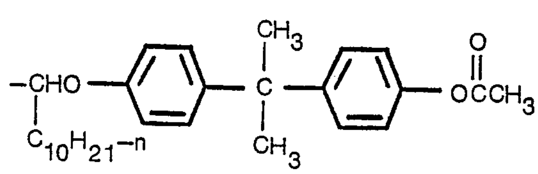 |

TABLE I (Continued)

| Coupler Number | X | R |
|---|---|---|
| 15 | –H | 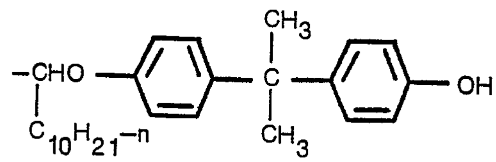 |
| 16 | –H | 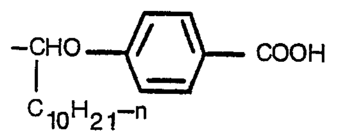 |
| 17 | 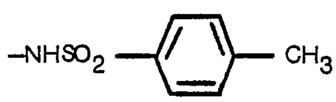 | 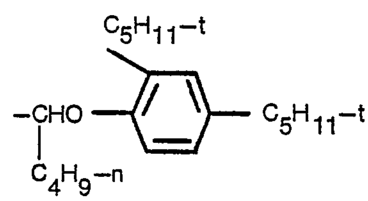 |

Couplers of this invention can be prepared by reacting *p*-cyanophenylisocyanate with an appropriate aminophenol, such as 2-amino-5-nitrophenol or 2-amino-4-chloro-5-nitrophenol to form the 2-(*p*-cyanophenyl) ureido compound. The nitro group can then be reduced to an amine, and the ballast group attached thereto by conventional procedures. Additional two equivalent couplers can be prepared by known techniques, for example, by substitution of the 4-chloro group on the starting phenol reactant. Examples 1 and 2 show the preparation of representative couplers.

The cyan dye-forming couplers of this invention can be used in the ways and for the purposes that cyan dye-forming couplers are used in the photographic art.

Typically, the coupler is incorporated in a silver halide emulsion and the emulsion coated on a support to form a photographic material. Alternatively, the coupler can be incorporated in a photographic material in a location adjacent to the silver halide emulsion layer where, during development, the coupler can react with oxidized colour developing agent. Both these methods of coupler incorporation are referred to when it stated hereinafter that a coupler is contiguous to a silver halide emulsion.

The photographic materials can be single colour materials or multicolour materials. In a multicolour material, the cyan dye-forming coupler of this invention is usually contiguous to a red-sensitive emulsion, although it can be contiguous to an unsensitized emulsion or an emulsion sensitized to a different region of the spectrum. Multicolour materials contain dye image-forming units sensitive to each of the three primary regions of the spectrum. Each unit can be comprised of a single emulsion layer or of multiple emulsion layers sensitive to a given region of the spectrum. The layers of the material, including the layers of the image-forming units, can be arranged in various orders as known in the art. In an alternative format, the emulsions sensitive to each of the three primary regions of the spectrum can be disposed as a single segmented layer, e.g., as by the use of microvessels laterally spaced on the surface of a support.

A typical multicolour photographic material comprises a support bearing a cyan dye image-forming unit comprising at least one red-sensitive silver halide emulsion layer contiguous to at least one cyan dye-forming coupler, at least one of the cyan dye-forming couplers being a coupler of this invention, a magenta dye image-forming unit comprising at least one green-sensitive silver halide emulsion layer contiguous to at least one magenta dye-forming coupler and a yellow dye image-forming unit comprising at least one blue-sensitive silver halide emulsion layer contiguous to at least one yellow dye-forming coupler. The material can contain additional layers, such as filter layers, interlayers, overcoat layers, and subbing layers.

In the following discussion of suitable constituents for use in the emulsions and materials of this invention, reference is made to the publication effected in *Research Disclosure,* December 1978, as Item 17643. For brevity this publication is referred to hereinafter as 'Research Disclosure'.

The silver halide emulsions employed in the photographic materials of this invention can be either negative-working or positive-working. Suitable emulsions and their preparation are described in Research Disclosure Sections I and II and the publications cited therein. Suitable vehicles for the emulsion layers and other layers of materials of this invention are described in Research Disclosure Section IX and the publications cited therein.

In addition to the couplers of this invention, the materials of the invention can include additional couplers as described in Research Disclosure Section VII, paragraphs D, E, F and G and the publications cited therein. These couplers can be incorporated in the materials and emulsion as described in Research Disclosure Section VII, paragraph C and the publications cited therein.

The photographic materials of this invention or individual layers thereof, can contain brighteners (see Research Disclosure Section V), antifoggants and stabilizers (see Research Disclosure Section VI), antistain agents and image dye stabilizer (see Research Disclosure Section VII, paragraphs I and J), light absorbing and scattering materials (see Research Disclosure Section VIII), hardeners (see Research Disclosure Section XI), plasticizers and lubricants (see Research Disclosure Section XII), antistatic agents (see Research Disclosure Section XIII), matting agents (see Research Disclosure Section XVI) and development modifiers (see Research Disclosure Section XXI).

The photographic materials can be coated on a variety of supports as described in Research Disclosure Section XVII and the references described therein.

Photographic materials can be exposed to actinic radiation, typically in the visible region of the spectrum, to form a latent image as described in Research Disclosure Section XVIII and then processed to form a visible dye image as described in Research Disclosure Section XIX. Processing to form a visible dye image includes the step of contacting the material with a colour developing agent to reduce developable silver halide and oxidize the colour developing agent. Oxidized colour developing agent in turn reacts with the coupler to yield a dye.

Suitable colour developing agents are aromatic primary amines such as p-phenylene diamines. Preferred colour developing agents include 4-amino-N,N-diethylaniline hydrochloride, 4-amino-3-methyl-N-ethyl-N-β-(methanesulphonamido)ethylaniline sulphate hydrate, 4-amino-3-methyl-N-ethyl-N-β-hydroxyethylaniline sulphate, 4-amino-3-β-(methanesulphonamido)ethyl-N,N-diethylaniline hydrochloride and 4-amino-N-ethyl-N-(2-methoxyethyl)-m-toluidine di-p-toluene sulphonic acid.

With negative-working silver halide, colour development leads to a negative image. To obtain a positive (or reversal) image, colour development can be preceded by black-and-white development with a non-chromogenic developing agent to develop exposed silver halide, but not form dye, the material being uniformly fogged to render the initially unexposed silver halide developable. Alternatively, a direct-positive emulsion can be employed to obtain a positive image colour image on colour development.

Development is followed by the conventional steps of bleaching, fixing or bleach-fixing, to remove silver and silver halide, washing and drying.

The following examples further illustrate this invention.

Example 1 — Preparation of Coupler No. 7

A. *Preparation of 2-(p-cyanophenyl)ureido-5-nitrophenol*

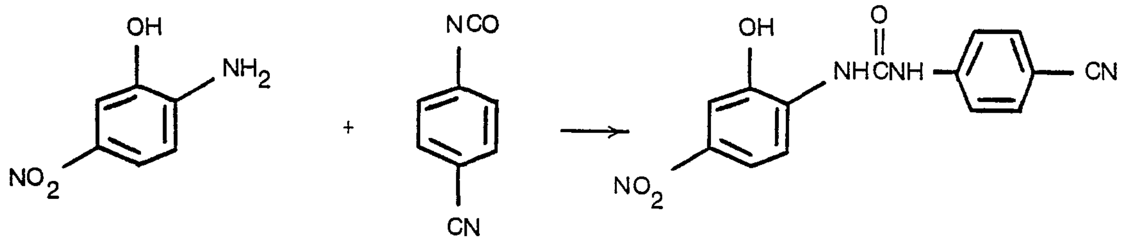

To a refluxing suspension of 23.1 g (0.15 mol) of 2-amino-5-nitrophenol in 300 ml. toluene was added with stirring a solution of 21.6 g (0.15 mol) p-cyanophenylisocyanate in 150 ml. toluene. The resulting reaction mixture was refluxed for 1 hour. After cooling the yellow precipitate was collected, washed with hot toluene and then with dioxane to give 41 g (92% yield) of product; m.p. 262—263°C.

B. *Preparation of Coupler No. 7*

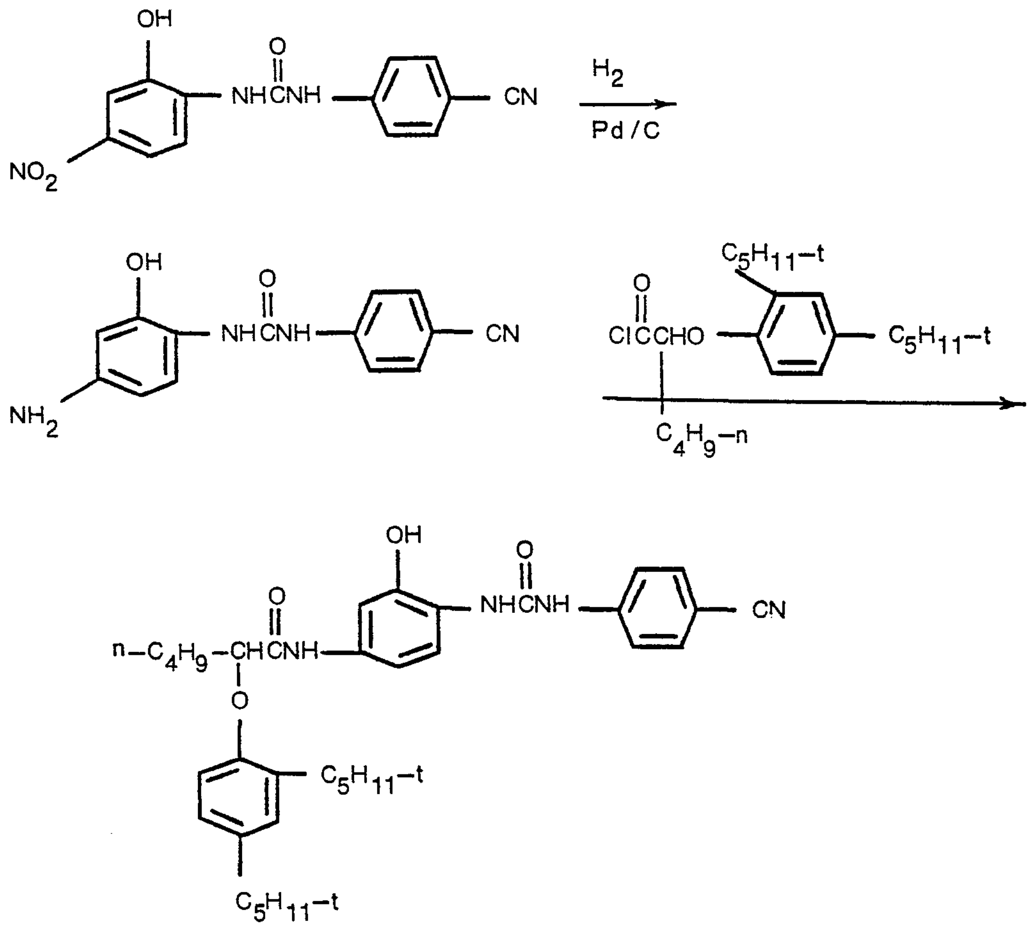


A suspension of 8.9 g (0.03 mol) of 2-(p-cyanophenyl)ureido-5-nitro phenol in 200 ml. tetrahydrofuran was catalytically reduced with a palladium on carbon catalyst under 276 kpa of hydrogen. After the theoretical uptake of hydrogen, the mixture was blanketed with nitrogen gas as 7.8 g. (0.06 mol) of quinoline and 10.9 g. (0.03 mol) of 2-(2,4-di-tert-pentyl phenoxy) hexanoyl chloride was added with stirring. The reaction mixture was stirred overnight at 22°C. The mixture was filtered to remove the catalyst. The filtrate was poured into ice-water containing 5 ml. of concentrated hydrochloric acid, extracted with diethyl ether, dried over magnesium sulphate and concentrated under reduced pressure to give a gummy white solid and recrystallized from toluene to give 11.4 g (63% yield) of white crystalline solid; m.p. 177—179°C.

Example 2 — Preparation of Coupler No. 11

A. *Preparation of 2-(p-cyanophenyl)ureido-4-chloro-5-nitrophenol*

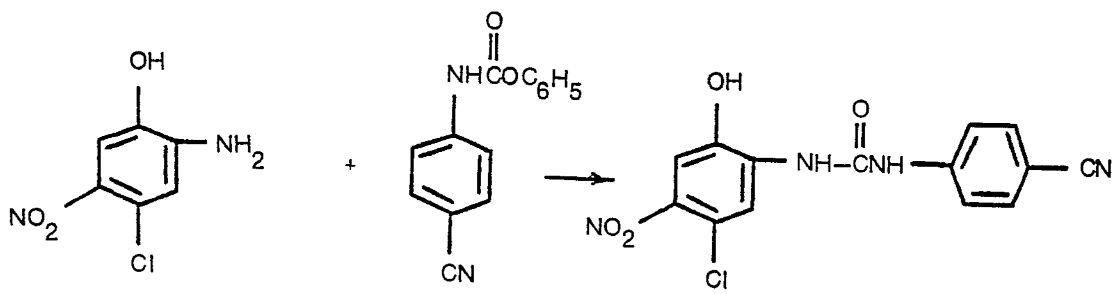

In a 1-litre flask was placed 37.6 g. (0.2 mol) of 2-amino-4-chloro-5-nitrophenol, 47.2 g. (0.2 mol) of phenyl p-cyanophenylcarbamate and 1.36 g. of imidazole in 400 ml. of xylene. The mixture was

heated to reflux for 5 hours. The brown solid was collected, washed with xylene and then with hexane to give 64 g. (97% yield) brown solid; m.p. 227—228°C.

B. *Preparation of Coupler No. 11*

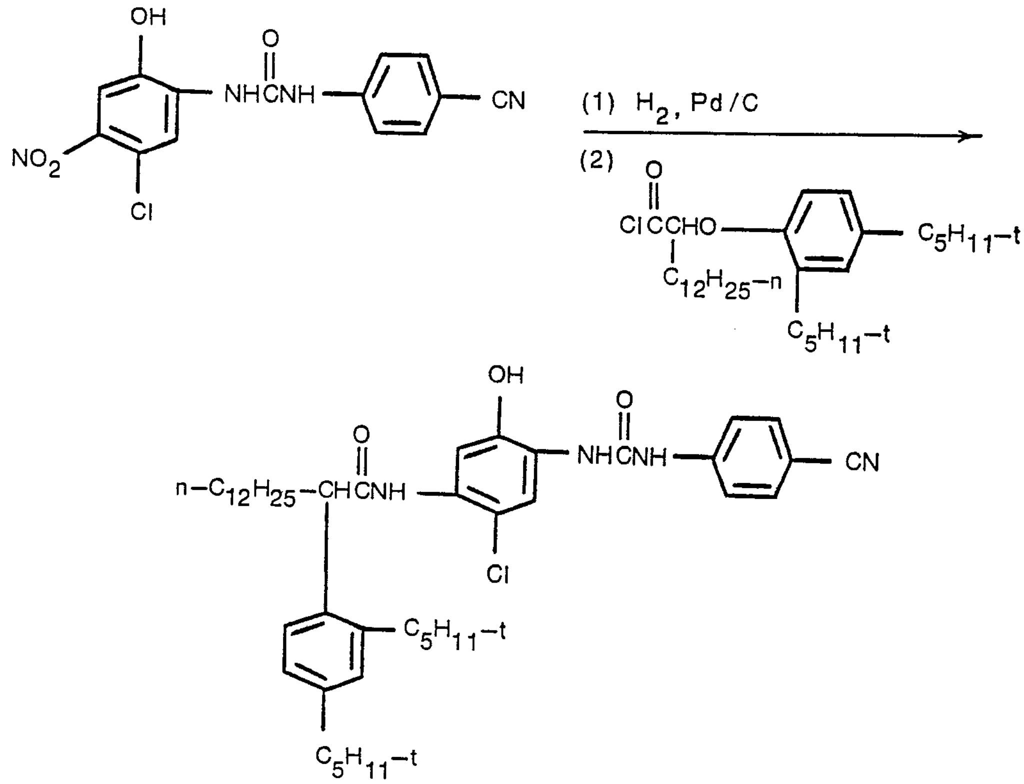


A suspension of 12 g. (0.036 mol) of 2-(p-cyanophenyl)ureido-4-chloro-5-nitrophenol and a teaspoonful of a palladium on carbon catalyst in 250 ml. of tetrahydrofuran was reduced under 276 kpa of hydrogen. After the theoretical uptake of hydrogen, nitrogen was allowed to bubble in. With stirring, 10 g. of quinoline was added followed by 17.2 g. (0.036 mol) of 2-(2,4-di-tert-pentylphenoxy) tetradecanoyl chloride in 40 ml. of tetrahydrofuran. The reaction mixture was stirred at 22°C for 3 hours and then filtered to remove the catalyst. The filtrate was poured into ice-water containing 10 ml. concentrated hydrochloric acid. The oil which separated was extracted with ethyl acetate dried over magnesium sulphate and concentrated under reduced pressure to give 28 g of residue. This residue was dissolved in methylene chloride and chromatographed through a silica gel column eluting with methylene chloride. The fractions containing the product were combined, concentrated under reduced pressure, and the residue recrystallized from acetonitrile to 11.4 g. of solid; m.p. 196—198°C.

Example 3

Photographic materials were prepared by coating a cellulose acetate film support with a light sensitive layer comprising a negative-working silver bromoiodide emulsion at 1.46 g Ag/m², gelatin at 4.86 g/m² and one of the following phenolic couplers at $1.68 \times 10^{-3}$ moles/m² dissolved in one half its weight of di-*n*-butylphthalate.

*Coupler*

Material A (control):

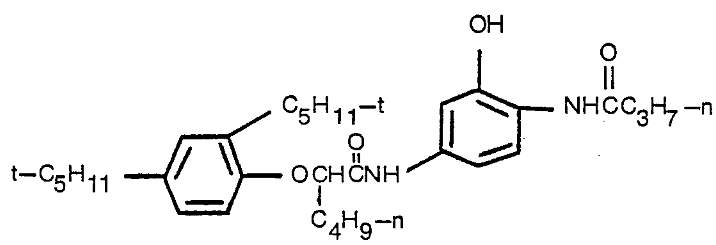

Material B: Coupler No. 7

Samples of each photographic material were sensitometrically exposed through a graduated-density test object and processed at 20°C by color developing for 10 minutes with the following developing solution; then stopped, washed, bleached, washed, fixed and washed.

*Colour Developer:*

| | | |
|---|---|---|
| Sodium hexametaphosphate | 0.5 | g |
| Benzyl alcohol | 4.0 | ml |
| $Na_2SO_3$ | 2.0 | g |
| $Na_2CO_3 \cdot H_2O$ | 50.0 | g |
| NaOH (40% solution) | 0.4 | ml |
| NaBr (50% solution) | 1.72 | ml |
| 4-Amino-3-methyl-N-ethyl-N-$\beta$-(methanesulphonamido) ethylaniline sulphate hydrate | 5.0 | g |

Water to 1 litre, pH 10.75.

Well-defined cyan dye images were produced. Sensitometric evaluation produced the results set out in Table II.

TABLE II

| Material | $D_{max}$ | $\lambda_{max}$ |
|---|---|---|
| A | 2.67 | 657 nm |
| B | 2.73 | 689 nm |

Example 4

Photographic materials were prepared by coating on a cellulose acetate film support a light-sensitive layer comprising a negative-working silver bromoiodide emulsion at a coverage dependent on coupler equivalency (see below), gelatin at 3.78 g/m², and a phenolic coupler (see below) at $1.62 \times 10^{-3}$ moles/m² dissolved in one half its weight of di-*n*-butylphthalate.

Samples of the materials were sensitometrically exposed through a graduated-density test object and processed at 20°C as described in Example 3, except colour development was for 2 minutes in the following developing solution:

| | | |
|---|---|---|
| $K_2SO_3$ | 2.0 | g |
| $K_2CO_3$ (anhydrous) | 30.0 | g |
| KBr | 1.25 | g |
| KI | 0.6 | mg |
| 4-Amino-3-methyl-N-ethyl-N-$\beta$-hydroxyethylaniline sulphate | 3.55 | g |

Water to 1.0 lit, pH 10.0.

Well-defined cyan dye images were produced which were sensitometrically evaluated. The results are recorded in Table III.

TABLE III

| Material | Coupler No. | Ag Coverage (g/m²) | $D_{max}$ | $D_{min}$ | $\lambda_{max}$ |
|---|---|---|---|---|---|
| C | 7 | 0.91 | 2.97 | 0.09 | 690 |
| D | 11 | 0.46 | 2.72 | 0.06 | 685 |

Example 5

A series of photographic materials was prepared, exposed and processed as described in Example 3, above, using the couplers identified below. Well-defined cyan dye images were produced. From the developed images spectrophotometric curves having a maximum density of 1.0 were generated and the wavelength of maximum dye density ($\lambda_{max}$) and the half-band width (HBW) were determined. Half-band width is the width in nanometers of the spectrophotometric curve at one half the difference between maximum density and dye fog. It is a measure of the purity of the hue of the dye; the narrower half-band width, the purer the hue.

The following results were obtained.

TABLE IV

| Material | Coupler | $\lambda$ max (nm) | HBW (nm) |
|---|---|---|---|
| F | 1 | 664 | 127 |
| G | 7 | 688 | 142 |
| H | A* | 668 | 148 |
| I | B* | 659 | 159 |
| J | C* | 644 | 137 |

*Coupler A is coupler 33 from U.S. Patent 3,880,661 and has the structure:

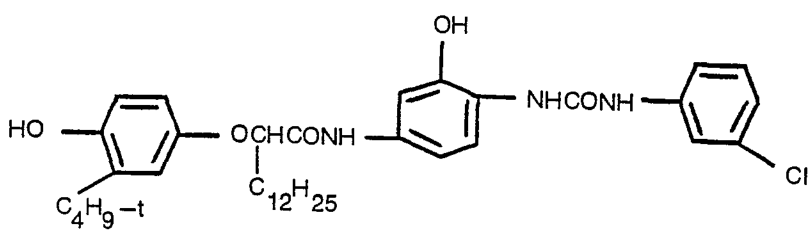

Coupler B is compound IV from U.S. Patent 3,758,308 and has the structure:

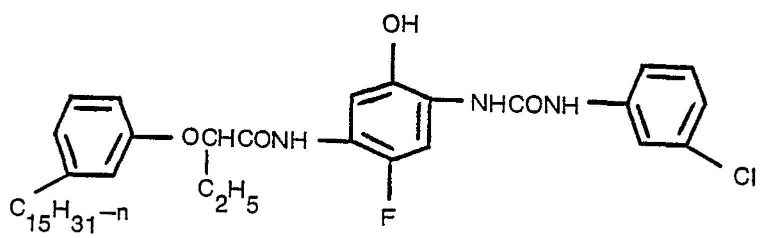

Coupler C is a non-ureido coupler having the structure:

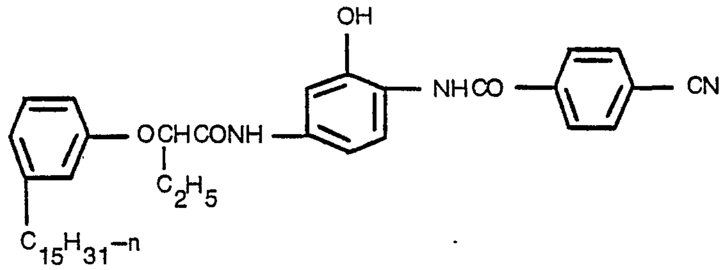

The data in Table IV indicate that dyes derived from couplers of the present invention absorb at longer wavelengths and/or have narrower half-band width than dyes derived from structurally close couplers which do not contain a p-cyanophenylureido group. Thus, the dyes from the present couplers have desirable and relatively pure absorption in the red region of the spectrum.

Example 6

A series of photographic materials was prepared, exposed and processed as described in Example 4, above, using the couplers identified below. Well-defined cyan dye images were obtained. From the developed images spectrophotometric curves having a maximum density of 1.0 were generated, from which $\lambda$ max and HBW were determined.

The results are reported in Table V.

TABLE V

| Element | Coupler | $\lambda$ max (nm) | HBW |
|---|---|---|---|
| N | 7 | 690 | 141 |
| O | D* | 694 | 177 |

*Coupler D is coupler VII from U.S. Patent 3,996,253 and has the structure:

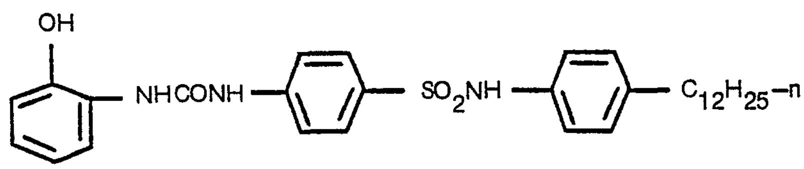

The data in Table V indicates that the dye from Coupler 7 has a narrower half-band width than the prior art dye and thus is sharper cutting dye of purer hue.

Example 7

Dye stability toward reduction by ferrous ions was determined by treating a processed sample of each of Materials N and O from Example 6 in a ferrous ion-containing solution in the following manner. A suspension was prepared by adding 32.12 g (0.11 mole) ethylenediamine tetraacetic acid to 750 ml concentrated ammonium hydroxide. To this suspension was added 27.8 g (0.10 mole) of ferrous sulphate heptahydrate with stirring and then concentrated ammonium hydroxide was slowly added until solution was obtained. The pH was then adjusted to 5.0 with nitric acid.

The samples were immersed in this ferrous ion-containing solution for 5 minutes at 20°C, washed in water for 5 minutes and dried. When compared to untreated samples, the following results were obtained.

| | $D_{max}$ | | |
|---|---|---|---|
| Material | Untreated | Treated | % Dye Loss |
| N | 2.16 | 2.08 | 3.7% |
| O | 2.30 | 0 | 91.7% |

Example 8

Photographic materials containing additional couplers of this invention were prepared, exposed and processed as in Example 4 and then spectrophotometric curves were generated and $\lambda_{max}$ and HBW were determined as in Example 5. The results are shown in Table VI.

TABLE VI

| Coupler | max | HBW |
|---|---|---|
| 5 | 675 | 136 |
| 6 | 655 | 153 |
| 8 | 691 | 135 |
| 9 | 674 | 151 |
| 11 | 695 | 137 |
| 12 | 691 | 133 |
| 13 | 685 | 141 |
| 14 | 684 | 133 |
| 15 | 681 | 136 |
| 16 | 669 | 152 |

The data in Table VI indicates that couplers of this invention, as a class, yield dyes having absorption maxima in the longer wavelength portion of the red region of the visible spectrum and generally have relatively narrow half-band widths. Thus, the dyes have little or no absorption in the green region of the spectrum and have relatively pure hues that are desirable for photographic images.

**Claims**

1. A photographic cyan dye-forming coupler having the structural formula:

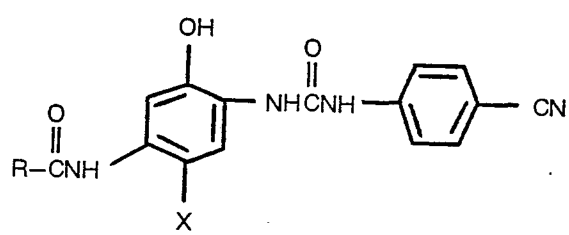

(I)

wherein:

X is a hydrogen or a coupling-off group; and

R is a ballast group.

2. A photographic cyan dye-forming coupler of Claim 1 wherein R has the structure:

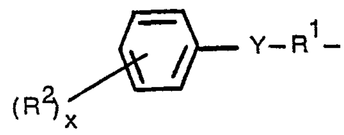

wherein:

Y is oxygen or sulphur;

$R^1$ is an alkylidene group of 2 to 20 carbon atoms having the formula

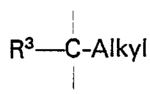

wherein $R^3$ is hydrogen or alkyl;

$R^2$ is hydroxy, carboxy, alkyl, aryl, aralkyl, alkoxyl, aryloxy, alkylsulphamoyl, arylsulphamoyl, alkylsulphonamido, arylsulphonamido, alkylsulphonyl, arylsulphonyl, alkoxycarbonyl, or acyloxy wherein alkyl contains 1 to 20 carbon atoms and aryl contains 6 to 20 carbons and wherein alkyl, aryl and aralkyl can be optionally substituted with hydroxy, carboxy, alkoxycarbonyl or acyloxy; and

x is 1 to 3.

3. A coupler according to Claim 1 wherein R of the formula I is:

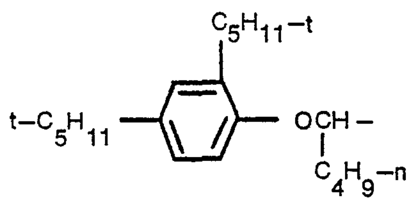

or

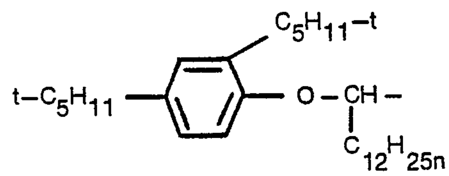

4. A photographic silver halide emulsion containing a cyan dye-forming coupler as specified in any of Claims 1 to 3.

5. A photographic recording material comprising a support and a photographic silver halide emulsion layer and incorporated in that layer or a layer adjacent thereto a cyan dye-forming coupler characterised in that the coupler is a coupler as specified in any of Claims 1 to 3.

6. A photographic process for forming a cyan dye image which comprises forming an imagewise distribution of developable silver halide in a photographic material and developing said distribution with a developing composition containing an aromatic primary amino colour developing agent characterised in the use of the photographic recording material according to claim 5.

**Patentansprüche**

1. Photographischer Blaugrünkuppler mit der Strukturformel

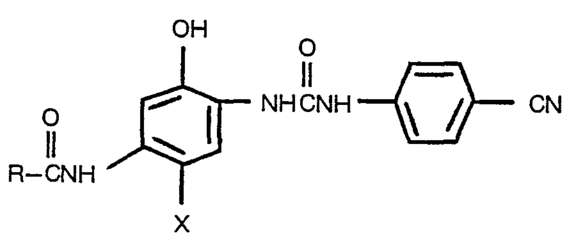

(I)

worin bedeuten:

X Wasserstoff oder eine abkuppelnde Gruppe; und

R eine Ballastgruppe.

2. Photographischer Blaugrünkuppler nach Anspruch 1, dadurch gekennzeichnet, daß R der Formel

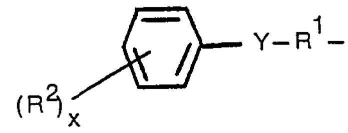

entspricht,
worin bedeuten:

Y Sauerstoff oder Schwefel;

$R^1$ eine Alkylidengruppe mit 2 bis 20 Kohlenstoffatomen, welche der Formel

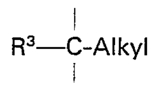

entspricht, worin $R^3$ Wasserstoff oder einen Alkylrest bedeutet;

$R^2$ eine Hydroxy-, Carboxy-, Alkyl-, Aryl-, Aralkyl-, Alkoxyl-, Aryloxy-, Alkylsulfamoyl-, Arylsulfamoyl-, Alkylsulfonamido-, Arylsulfonamido-, Alkylsulfonyl-, Arylsulfonyl-, Alkoxycarbonyl- oder Acyloxygruppe, wobei die Alkylgruppe 1 bis 20 und die Arylgruppe 6 bis 20 Kohlenstoffatome enthält, und wobei die Alkyl-, Aryl- und Aralkylgruppe gegebenenfalls durch eine Hydroxy-, Carboxy-, Alkoxycarbonyl- oder Acyloxygruppe substituiert sein kann; und

X 1 bis 3.

3. Kuppler nach Anspruch 1, dadurch gekennzeichnet, daß R in der Formel I

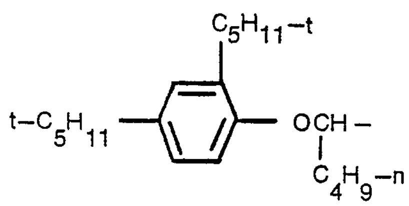

oder

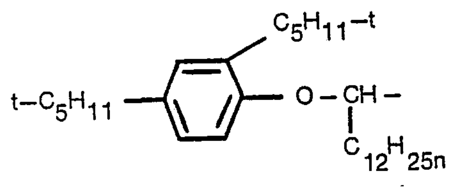

entspricht.

4. Photographische Silberhalogenidemulsion, die einen Blaugrünkuppler nach einem der Ansprüche 1 bis 3 enthält.

5. Photographisches Aufzeichnungsmaterial, welches einen Träger und eine photographische Silberhalogenidemulsionsschicht aufweist, wobei in dieser Schicht oder einer dieser Schicht benachbarten Schicht ein Blaugrünkuppler enthalten ist, dadurch gekennzeichnet, daß der Kuppler ein wie in den Ansprüchen 1 bis 3 beschriebener Kuppler ist.

6. Photographisches Verfahren zur Erzeugung eines blaugrünen Farbstoffbildes, welches folgende Schritte umfaßt:

— Erzeugung einer bildmäßigen Verteilung von entwickelbarem Silberhalogenid in einem photographischen Material und
— Entwicklung des bildmäßig verteilten Silberhalogenids mit einer Entwicklermasse, die eine Farbentwicklerverbindung aus einem aromatischen primären Amin enthält,

gekennzeichnet durch die Verwendung des photographischen Aufzeichnungsmaterials nach Anspruch 5.

**Revendications**

1. Coupleur photographique formateur de colorant bleu-vert correspondant à la formule suivante:

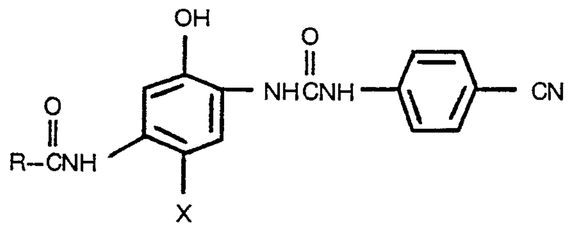

où:

X représente un atome d'hydrogène ou un groupement se séparant au couplage, et,
R représente un groupe de lestage (ou groupe ballast).

2. Coupleur conforme à la revendication 1, où R présente la structure suivante:

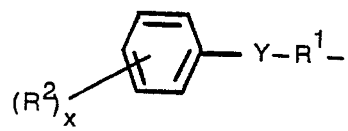

où:

Y représente un atome d'oxygène ou de soufre,
$R^1$ représente un groupe alkyldène de 2 à 20 atomes de carbone répondant à la formule:

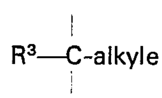

où:

$R^3$ représente un atome d'hydrogène ou un groupe alkyle,
$R^2$ représente un groupe hydroxy, carboxy, alkyle, aryle, aralkyle, alkoxyle, aryloxy, alkylsulfamoyle, arylsulfamoyle, alkylsulfonamido, arylsulfonamido, alkylsulfonyle, arylsulfonyle, alkoxycarbonyle, ou acyloxy où chaque groupe alkyle contient 1 à 20 atomes de carbone et chaque groupe aryle contient 6 à 20 atomes de carbone et où chaque groupe alkyle, aryle et aralkyle peut être éventuellement substitué par un groupe hydroxyle, carboxy, alkoxycarbonyle ou acyloxy, et,
x est égal à 1, 2 ou 3.

3. Coupleur conforme à la revendication 1, où R de la formule I représente les groupements:

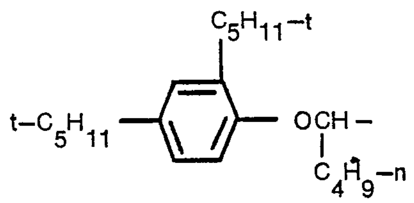

ou

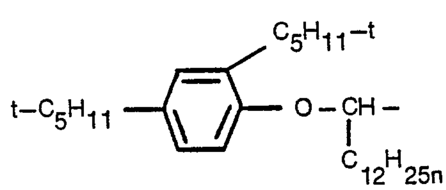

4. Emulsion photographique aux halogénures d'argent qui contient un coupleur formateur de colorant bleu-vert conforme à l'une quelconque des revendications 1 à 3.

5. Produit d'enregistrement photographique qui comprend un support, une couche d'émulsion photographique aux halogénures d'argent et un coupleur, formateur de colorant bleu-vert, incorporé dans cette couche ou dans une couche adjacente, ce produit étant caractérisé en ce que le coupleur est un coupleur conforme à l'une quelconque des revendications 1 à 3.

6. Procédé photographique pour former une image de colorant bleu-vert, qui consiste à former, suivant une image une répartition d'halogénures d'argent développables dans un produit photographique puis à développer cette répartition par un révélateur qui contient un développateur chromogène à fonction amine aromatique primaire, ce procédé étant caractérisé en ce qu'on utilise le produit d'enregistrement photographique conforme à la revendication 5.